# EUROPEAN PATENT SPECIFICATION

(11) **EP 1 372 693 B1**
(45) Date of publication and mention of the grant of the patent: **14.06.2006**
(21) Application number: 02706935.0
(22) Date of filing: 26.03.2002
(51) Int. Cl.: A61K 38/07, C07K 5/10, A61P 17/02

(54) **PEPTIDES FOR THE TREATMENT OF WOUND CONTRACTURE**
PEPTIDE ZUR BEHANDLUNG VON WUNDKONTRAKTION
PEPTIDES UTILISES POUR LE TRAITEMENT DU PHENOMENE DE CONTRACTURE D'UNE PLAIE

(30) Priority: 28.03.2001 GB 0107761
(43) Date of publication of application: 02.01.2004
(73) Proprietor: Johnson & Johnson Medical Ltd., Edinburgh EH2 4NH (GB)
(72) Inventor: CULLEN, Breda, Mary, North Yorkshire BD23 1HR (GB); SILCOCK, Derek, Walter, North Yorkshire BD23 1QP (GB)
(74) Representative: James, Anthony Christopher W.P.
(86) International application number: PCT/GB2002/001173
(87) International publication number: WO 2002/078728

(56) References cited:
- EP-A- 0 858 808
- GB-A- 2 314 842
- US-A- 4 957 902
- W. B. SAUNDERS CO "WOUND HEALING: BIOCHEMICAL & CLINICAL ASPECTS", I. KELMAN COHEN ET AL. EDS. R RUDOLPH ET AL " Wound contraction and scar contracture", pages 96-114 * the whole document *

## Description

The present invention relates to the use of certain peptides for the preparation of medicaments for use in the treatment or prevention of wound contracture, in particular the prevention of burn contracture.

Wound contraction is the process which diminishes the size of a full-thickness open wound, especially a full-thickness burn. The tensions developed during contracture and the formation of subcutaneous fibrous tissue can result in deformity, and in particular to fixed flexure or fixed extension of a joint where the wound involves an area over the joint. Such complications are especially prevalent in burn healing.

Scar contracture is the result of the contractile process occurring in a healed scar, and often results in an undesirable, fixed, rigid scar that can cause functional or cosmetic deformity. In general, wound contraction occurs in an incompletely epithelialised defect, while scar contracture occurs in an epithelialised covered defect. The term "wound contracture" is used herein to cover both wound contraction and scar contracture.

Wound contraction and scar contracture are reviewed in detail by Ross Rudolph, Jerry Vande Berg and H. Paul Ehrlich in Wound Healing: Biochemical and Clinical Aspects, I.K. Cohen, R.F. Diegelmann and W.J. Lindblad Eds, W.B. Saunders Company, pages 96-114.

The deleterious effects of contracture can also occur underneath the skin surface or involve deep vital organs. Well-known examples are contraction of the palmar fascia in Dupuytren's contracture and the contraction of a capsule around a silicone breast implant, and certain complications of cardiac pacemaker implants. In addition, contracted fibrotic scar bundles within the cirrhotic liver may compress intrahepatic vessels and contribute to portal hypertension. Other viscera can undergo contractures, such as in duodenal ulcer stricture, the contracture of cardiac valves in rheumatic heart disease, and urethral strictures in trauma.
Fibroblasts and myofibroblasts have been implicated in the mechanism of wound contraction. It has been suggested to use substances or procedures which interfere with fibroblast and myofibroblast mobilisation, migration, adhesion or multiplication for the inhibition of wound contracture. For example, high doses of cortisone or related steroids have been shown to suppress fibroblast proliferation, and thereby inhibit wound contracture. Unfortunately, steroids given in such high doses give unacceptable side effects in clinical practice.

Cellular poisons such as cyanide and dinitrophenol, have also been reported to inhibit wound contraction. Likewise, drugs which inhibit smooth muscle contraction have been reported to inhibit wound contraction, for example, colchicine, vinblastine and phenyltoin.

In practice, control of wound contracture is presently mainly mechanical and surgical. The surgeon may wish to facilitate wound contraction in some situations and to inhibit it in others. The following approaches are used to reduce undesirable scar and wound contraction: (1) prolonged splinting, (2) range of motion exercises, (3) pressure, (4) full-thickness skin grafting, and (5) judicious planning of surgical procedures.

It has also been found that an adherent dressing, such as untreated gauze, can delay, but does not prevent contracture. In contrast, certain synthetic films such as nylon applied to the wound surface before active contracture has started can inhibit contracture.

It has been found that application of a full-thickness skin graft to an open wound before wound contracture commences is effective to prevent wound contracture. However, significant problems are associated with skin grafting including cost, the source of grafting skin, rejection of the graft, secondary infection, and associated surgical risks. Severe contracture is still observed when thin or meshed skin grafts are applied instead of full-thickness skin grafts.

US-A-4957902 describes the inhibition of wound contracture by application to the wound of peptides having an amino acid sequence Arg-Gly-Glu and N-terminal and C-terminal derivatives thereof.

It has now been found that certain other peptides display promising activity *in vitro* that is indicative of ability to prevent or reduce wound and scar contracture.

Accordingly, the present invention provides the use of a peptide or peptide derivative having the sequence X-NH-Gly-Pro-Ala-Gly-CO-Y, wherein X is H or a pharmaceutically acceptable N-terminal group, and Y is OH or a pharmaceutically acceptable C-terminal group, for the preparation of a medicament for use in the treatment or prevention of wound contracture.

Earlier patent application GB-A-2321191 describes the use of such peptides for the treatment of chronic wounds, such as diabetic ulcers.

It has now been found by means of the *in vitro* fibroblast populated collagen gel contraction model described in detail below under procedure 1 that the tetrapeptide Gly-Pro-Ala-Gly is surprisingly effective to inhibit collagen gel contraction. This effect was observed with both neonatal and foetal fibroblasts. It was further found that the tetrapeptide inhibited the stimulatory effect of certain growth factors on fibroblast populated collagen gel contraction. These results provide a clear indication that the tetrapeptides are likely to be effective to reduce wound contracture *in vivo.* Furthermore, these results indicate that a number of derivatives of the tetrapeptide when used as a dressing are likely to reduce wound contracture, either due to analogous activity or becouse the derivative breaks down gradually *in vivo* in the wound bed to the tetrapeptide.

Preferably, X is a pharmaceutically acceptable N-terminal group that is hydrolyzed or otherwise metabolized to H under physiological conditions. Preferably, Y is a pharmaceutically acceptable C-terminal group that is hydrolyzed or otherwise metabolized to OH under physiological conditions.

For example, X may be a C1-C8 alkyl group or a C1-C8 alkylcarbonyl group. In other preferred embodiments, X may be an amino acid or a short peptide chain, provided that the total peptide length of the active compound is no more than 12 amino acids, preferably no more than 6 amino acids.

Likewise, Y may be a C1-C8 alkoxy group or a C1-C8 alkylamino group. In other preferred embodiments, X may be an amino acid or a short peptide chain, provided that the total peptide length of the active compound is no more than 12 amino acids, preferably no more than 6 amino acids.

Preferably, X is H and Y is OH, i.e., the tetrapeptide is not derivatized.

The definition of the active compound given herein encompasses all active salts and stereoisomers thereof.

Preferably, the medicament contains from 0.0002% to 10% by weight of the peptide or peptide derivative, more preferably from 0.0005% to 1%, still more preferably from 0.001 % to 0.1%, and most preferably from 0.002% to 0.01%.

Preferably, in the use according to the present invention, the medicament comprises a solid wound dressing for topical application. For example, the tetrapeptide or derivative thereof may be dispersed in or on a solid wound dressing material such as a woven or nonwoven fabric, a film or a sponge that is applied to the surface of the wound to reduce contracture.

The wound dressing material may be a material that is absorbable *in vivo.* For example, a synthetic or semi-synthetic polymer such as polylactide/polyglycolide, or oxidized regenerated cellulose (ORC). The material may comprise a biopolymer such as collagen, gelatin, an alginate, chitosan, hyaluronic acid, guar gum or xanthan gum. In certain preferred embodiments of the present invention, the peptide is dispersed in a matrix of oxidized cellulose complexed with collagen to form structures of the kind described in WO98/00180 and WO98/00446, the entire contents of which are expressly incorporated herein by reference. For example, the oxidized cellulose may be in the form of milled ORC fibres that are dispersed in a freeze-dried collagen sponge. This provides for sustained release of the oxidized cellulose to the wound, together with certain therapeutic and synergistic effects arising from the complexation with collagen. Finally, ORC itself has the property of reducing wound contracture, as demonstrated in GB-A-2354708.

In other preferred embodiments, the use according to the present invention provides a medicament in the form of an ointment or gel for topical application to a wound comprising the active material in a pharmaceutically acceptable carrier. Suitable carriers include: hydrogels containing cellulose derivatives, including hydroxyethyl cellyulose, hydroxymethyl cellulose, carboxymethyl cellulose, hydroxypropyl methyl cellulose, and mixtures thereof, and hydrogels containing polyacrylic acid (Carbopols). Suitable carriers also include creams and ointments used for topical pharmaceutical preparations, e.g. creams based on cetomacrogol emulsifying ointment. The carriers may include alginate (as a thickener or stimulant), preservatives such as benzyl alcohol, buffers to control pH such as disodium hydrogen phosphate/ sodium dihydrogen phosphate, agents to adjust osmolarity such as sodium chloride, and stabilizers such as ethylene diamine tetra acetate (EDTA).

The dressings comprising the peptide may be sterilized, for example by gamma-irradiation, and packaged in a microorganism-impermeable container.

The medicaments provided by the present invention may be applied to a wound or a burn shortly after the wound or burn has been inflicted.

Preferably, the medicaments provided by the present invention are applied from two to seven days after the wound or burn has been inflicted, since this is when contracture of the wound or burn is thought to start. In other preferred embodiments the medicaments provided by the present invention are applied to the wound or burn after epithelialisation is complete, for the reduction of scar contracture. Preferably, the medicaments provided by the present invention are applied both before and after epithelialisation of the wound is complete. Preferably, the application is continued for at least one week, more preferably at least two weeks, still more preferably at least four weeks, and most preferably six weeks or more. This reflects the fact that the tissue remodelling results in wound contraction and scar contracture is a longer-term process that typically begins some time after the onset of wound healing, and continues after epithelialisation of the wound is complete.

Preferably, the wound is a granulating wound. Preferably, the wound is a full-thickness wound or burn. Preferably, the full thickness area of the wound is at least 5cm², more preferably at least 10cm², and most preferably at least 20cm². Preferably, the wound is a burn. The medicaments are also expected to be useful for the reduction of contracture in the region of breast implants or cardiac pacemaker implants.

It will be appreciated that the present application can also be useful in the treatment of internal wounds. Administration may be by any means that facilitate the contracture-inhibiting effect of the peptide or peptide derivative. Preferably, the administration is topical. Preferably, the medicament is manufactured in accordance with any of the preferred embodiments of the use according to the invention, as enumerated above.

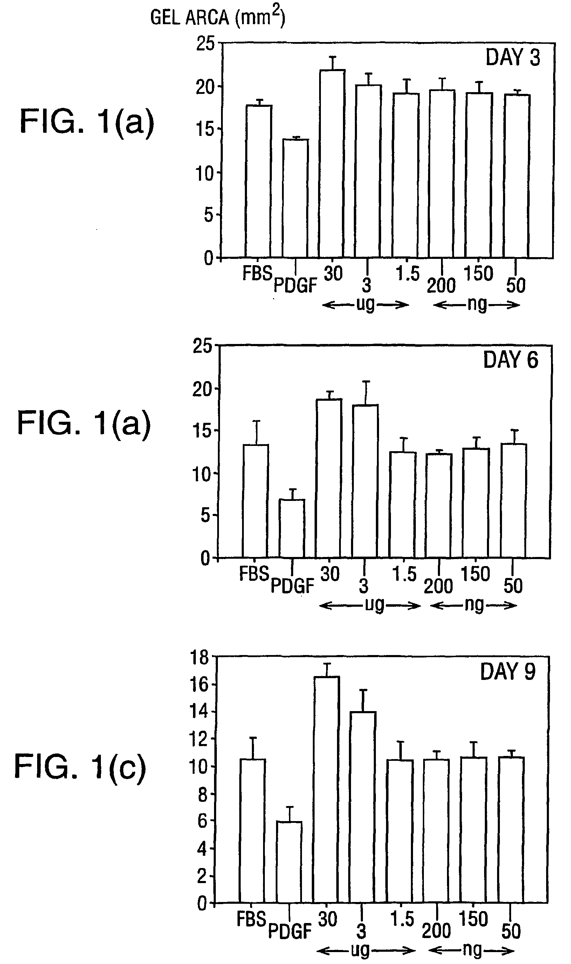

Particular embodiments of the present invention will now be described further, by way of example, with reference to the accompanying drawings, in which:-
Figure 1 illustrates the effect various concentrations of the tetrapeptide Gly-Pro-Ala-Gly on dermal fibroblast populated collagen gel contraction, the measurements being carried out in the presence of 10ng/ml of platelet derived growth factor (PDGF), (a) at time T=day 3, (b) at time T=day 6, and (c) at time T=day 9.

### Procedure 1

The inhibitory effect of the tetrapeptide Gly-Pro-Ala-Gly on fibroblast populated collagen gel contraction was determined by a method similar to that described in US-A-4957902.

Briefly, fibroblast populated collagen gels were prepared as follows: neonatal fibroblasts (HSF 43 SK supplied by ATCC) were grown to confluency in 10% Fetal Bovine Serum (FBS)/Dulbecco's Modified Eagles Medium (DMEM). The cells were harvested using 0.05% trypsin/EDTA, counted and centrifuged to remove trypsin solution. The cells were resuspended at a cell density of 10⁶ cells/ml.

The following mixture was then made up: 8mls 10% FBS/DMEM, 4mls of the cell suspension, and 4mls of aqueous VITROGEN collagen supplied by Collagen Corporation, Palo Alto, CA (concentration 4mg/ml). The mixture was then distributed at 0.5ml/well in a 24-well plate and allowed to gel at 37°C for 1 hr.

Once the gels had set they were rimmed with a sterile pipette tip to enable them to contract freely, and then additional 0.5ml aliquots of FBS/DMEM were added carefully to each well. The aliquots additionally contained the following active ingredients:
None (negative control)
10 ng/ml Platelet Derived Growth Factor (PDGF, positive control)
30 µg tetrapeptide
3 µg tetrapeptide
1.5 µg tetrapeptide
200 ng tetrapeptide
150 ng tetrapeptide
5 ng tetrapeptide
When present, the tetrapeptide was prepared in accordance with Example 1 hereinbelow at a concentration of 0.5mg/ml, giving a final concentration in the gels of 0.165mg/ml. All solutions were sterile filtered. Each growth factor was incubated for 1hr at 37°C in the stock solution before its addition to the rimmed collagen gels.

Contraction of the collagen gels was measured by taking photographs of each gel over 10 days, and measuring the gel area from the photographs. The results can be summarised as follows, with reference to the Figures.

Referring to Figure 1(a), this bar chart shows the collagen gel area at t=3 days for the collagen gels populated with fibroblasts. It can be seen that all of the gels have shrunk significantly due to fibroblast proliferation in the gel. The test gels containing the tetrapeptide appear to have shrunk slightly less that the negative control gel, but this may not be statistically significant.

Referring to Fig. 1(b), this shows the areas of the gel samples of Fig. 1(a) at t=6 days. It can be seen that all of the gels have shrunk further due to fibroblast proliferation in the gel. It can also clearly be seen that the samples containing higher concentrations of the tetrapeptide exhibit a statistically significant reduction in contracture of the gel area after 6 days compared to the samples that contain less than 3 µg of the tetrapeptide. The same effect is even more marked at t=9 days, as shown in Fig. 1(c).

### Example 1

The tetrapeptide Gly-Pro-Ala-Gly was prepared in a fully automated Applied Biosystems 430A peptide synthsiser. The Fmoc/tBu based method of peptide synthesis was used, which involves the use of the base labile 9-fluorenylmethoxycarbonyl amino protecting group in conjunction with acid labile side protection and peptide-resin linkage. The peptide was synthesized using Fmoc-glycine functionalized 4-alkoxybenzyl alcohol resin (Wang Corporation), and all amino acids were incorporated using double coupling cycles. Each synthetic cycle involved: (1) treatment with acetic anhydride to cap any free amino groups prior to amine deprotection, (2) Fmoc removal by treatment with the organic base piperidine, and (3) coupling of the next amino acid in the sequence. In this way the desired peptide was built up from the C to N terminus. The peptide was then cleaved from the resin with simultaneous removal of side chain protecting groups by treatment with a mixture of TFA/ethanediol/triisopropylsilane/thioanisole and water for 3 hours at room temperature. The resin was then removed by filtration, the TFA evaporated and the peptide isolated by precipitation with diethyl ether and filtration. The crude peptide was then purified by reverse phase HPLC and lyophilized. Laser desorption mass spectroscopy and analytical HPLC were carried out to confirm purity of the peptide.

### Example 2

A solid, bioabsorbable collagen/ORC sponge dressing containing the peptide of example 1 is prepared as described in WO98/00180. Briefly, the freeze-dried collagen prepared as described in US-A-4614794 or US-A-4320201 is resuspended in 0.05m acetic acid at a concentration of 10mg/ml. Milled ORC powder (milled SURGICEL® cloth) is added to the suspension at a ratio of 1:3 ORC:collagen and homogenized using a Waring blender on low speed for 3x30 seconds. The peptide is then added to the suspension at a concentration of 1µg/ml. The complex suspension is degassed in a vacuum oven for 10 minutes, and is then poured to a depth of 3mm into a tray and blast frozen. The frozen suspension is then either freeze-dried and dehydrothermally cross-linked using a programmable freeze-drier with a temperature ramping facility, or it is dried using a solvent drying process as described in US-A-2157524. The dressing is suitable for application to a large-area burn to reduce contracture

### Example 3

A gel ointment suitable for application to wounds for the prevention of contracture is prepared according to the following formulation:-

| | |
|---|---|
| Carboxymethylcellulose | 2.4% |
| Hydroxyethylcellulose | 0.3% |
| Sodium chloride | 0.24% |
| Propylene glycol | 20.2% |
| Gly-Pro-Ala-Gly | 0.01% |
| Water | balance |

The sterile pharmaceutical gel was formulated under aseptic conditions.

The above embodiments have been described by way of example only.

## Claims

1. Use of a peptide or peptide derivative having the sequence X-NH-Gly-Pro-Ala-Gly-CO-Y, wherein X is H or a pharmaceutically acceptable N-terminal group, and Y is OH or a pharmaceutically acceptable C-terminal group, for the preparation of a medicament for use in the treatment or prevention of wound contracture.

2. Use according to claim 1, wherein X is a pharmaceutically acceptable N-terminal group that is hydrolyzed or otherwise metabolized to H under physiological conditions.

3. Use according to claim 1 or claim 2, wherein Y is a pharmaceutically acceptable C-terminal group that is hydrolyzed or otherwise metabolized to OH under physiological conditions.

4. Use according to any preceding claim, wherein X is a C1-C8 alkyl group or a C1-C8 alkylcarbonyl group or an amino acid.

5. Use according to any preceding claim, wherein Y is a C1-C8 alkoxy group or a C1-C8 alkylamino group or an amino acid.

6. Use according to claim 1, wherein X is H and Y is OH.

7. Use according to any preceding claim, wherein the medicament contains from 0.001 % to 1% by weight of the peptide or peptide derivative.

8. Use according to any preceding claim, wherein the medicament comprises a solid wound dressing having the peptide or peptide derivative dispersed thereon or therein.

9. Use according to claim 8, wherein said solid wound dressing comprises a solid material that is absorbable in vivo.

10. Use according to claim 9, wherein said solid material comprises oxidized cellulose complexed to collagen.

11. Use according to any preceding claim, wherein said wound contracture comprises burn contracture.

12. Use according to any preceding claim, wherein said medicament comprises an ointment or gel for application to a wound.

13. Use according to any preceding claim, wherein said treatment comprises application to the wound after epithelialisation is complete.

## Patentansprüche

1. Verwendung eines Peptids oder Peptidderivats, welches die Sequenz X-NH-Gly-Pro-Ala-Gly-CO-Y aufweist, wobei X H oder eine pharmazeutisch zulässige N-Endgruppe ist und Y OH oder eine pharmazeutisch zulässige C-Endgruppe ist, für die Zubereitung eines Medikaments zur Verwendung bei der Behandlung oder Vorbeugung von Wundkontraktur.

2. Verwendung nach Anspruch 1, **dadurch gekennzeichnet, daß** X eine pharmazeutisch zulässige N-Endgruppe ist, welche unter physiologischen Bedingungen zu H hydrolisiert oder auf andere Weise umgewandelt ist.

3. Verwendung nach Anspruch 1 oder Anspruch 2, **dadurch gekennzeichnet, daß** Y eine pharmazeutisch zulässige C-Endgruppe ist, welche unter physiologischen Bedingungen zu OH hydrolisiert oder auf andere Weise umgewandelt ist.

4. Verwendung nach einem der vorangehenden Ansprüche, **dadurch gekennzeichnet, daß** X eine C1-C8-Alkylgruppe oder eine C1-C8-Alkylcarbonylgruppe oder eine Aminosäure ist.

5. Verwendung nach einem der vorangehenden Ansprüche, **dadurch gekennzeichnet, daß** Y eine C1-C8-Alkoxilgruppe oder eine C1-C8-Alkylaminogruppe oder eine Aminosäure ist.

6. Verwendung nach Anspruch 1, **dadurch gekennzeichnet, daß** X H ist und Y OH ist.

7. Verwendung nach einem der vorangehenden Ansprüche, **dadurch gekennzeichnet, daß** das Medikament nach Gewicht zwischen 0,001 % bis 1% des Peptids oder des Peptidederivats umfaßt.

8. Verwendung nach einem der vorangehenden Ansprüche, **dadurch gekennzeichnet, daß** das Medikament eine feste Wundabdeckung umfaßt, auf welcher oder in welcher das Peptid oder das Peptidderivat verteilt ist.

9. Verwendung nach Anspruch 8, **dadurch gekennzeichnet, daß** die feste Wundabdekkung ein festes Material umfaßt, welches in vivo absorbierbar ist.

10. Verwendung nach Anspruch 9, **dadurch gekennzeichnet, daß** das feste Material oxidierte Zellulose umfaßt, welche zu Collagen zusammengesetzt sind.

11. Verwendung nach einem der vorangehenden Ansprüche, **dadurch gekennzeichnet, daß** die Wundkontraktur eine Brandkontraktur umfaßt.

12. Verwendung nach einem der vorangehenden Ansprüche, **dadurch gekennzeichnet, daß** das Medikament eine Salbe oder ein Gel zum Auftragen auf eine Wunde umfaßt.

13. Verwendung nach einem der vorangehenden Ansprüche, **dadurch gekennzeichnet, daß** die Behandlung ein Auftragen auf die Wunde umfaßt, nachdem Epithelialisation abgeschlossen ist.

## Revendications

1. Utilisation d'un peptide ou d'un dérivé peptidique ayant la séquence X-NH-Gly-Pro-Ala-Gly-CO-Y, dans laquelle X est un atome d'hydrogène ou un groupe N-terminal acceptable d'un point de vue pharmaceutique et Y est OH ou un groupe C-terminal acceptable d'un point de vue pharmaceutique, pour la préparation d'un médicament pour une utilisation dans le traitement ou la prévention d'une contracture d'une plaie.

2. Utilisation selon la revendication 1, dans laquelle X est un groupe N-terminal acceptable d'un point de vue pharmaceutique qui est hydrolysé ou autrement métabolisé en atome d'hydrogène dans des conditions physiologiques.

3. Utilisation selon la revendication 1 ou la revendication 2, dans laquelle Y est un groupe C-terminal acceptable d'un point de vue pharmaceutique qui est hydrolysé ou autrement métabolisé en OH dans des conditions physiologiques.

4. Utilisation selon l'une quelconque des revendications précédentes, dans laquelle X est un groupe alkyle en C₁ à C₈ ou un groupe alkyle carbonyle en C₁ à C₈ ou un acide aminé.

5. Utilisation selon l'une quelconque des revendications précédentes, dans laquelle y est un groupe alcoxy en C₁ à C₈ ou un groupe alkylamino en C₁ à C₈ ou un acide aminé.

6. Utilisation selon la revendication 1, dans laquelle X est un atome H et Y est OH.

7. Utilisation selon l'une quelconque des revendications précédentes, dans laquelle le médicament contient de 0,001 % à 1 % en poids du peptide ou dérivé peptidique.

8. Utilisation selon l'une quelconque des revendications précédentes, dans laquelle le médicament comprend un pansement pour plaie solide ayant le peptide ou dérivé peptidique dispersé sur celui-ci ou à l'intérieur de celui-ci.

9. Utilisation selon la revendication 8, dans laquelle ledit pansement pour plaie solide comprend un matériau solide qui est absorbable in vivo.

10. Utilisation selon la revendication 9, dans laquelle ledit matériau solide comprend de la cellulose oxydée complexée à du collagène.

11. Utilisation selon l'une quelconque des revendications précédentes, dans laquelle ladite contracture d'une plaie comprend une contracture d'une brûlure.

12. Utilisation selon l'une quelconque des revendications précédentes, dans laquelle ledit médicament comprend une pommade ou un gel pour une application à une plaie.

13. Utilisation selon l'une quelconque des revendications précédentes, dans laquelle ledit traitement comprend l'application à la plaie après achèvement de l'épithélisation.
